# EUROPEAN PATENT APPLICATION

(11) **EP 4 767 954 A1**
(43) Date of publication of application: **01.07.2026**
(21) Application number: 24855756.3
(22) Date of filing: 18.08.2024
(51) Int. Cl.: A61B 17/32, A61B 18/12

(54) **AUTOMATIC DETERMINATION METHOD AND SYSTEM FOR ULTRASOUND ABLATION BALLOON APPOSITION**

(30) Priority: 23.08.2023 CN 202311067935
(71) Applicant: Brattea Medtech Co., Ltd., Shanghai 201114 (CN)
(72) Inventor: GUO, Jiulin, Shanghai 200231 (CN); HUANG, Hanli, Shanghai 200231 (CN)
(74) Representative: Wang, Bo
(86) International application number: PCT/CN2024/112941
(87) International publication number: WO 2025/040033

(57) **Abstract**

Disclosed in the present invention are an automatic determination method and system for ultrasound ablation balloon apposition. The method comprises the following steps: setting related working parameters; after a balloon moves to a target ablation area, injecting normal saline; measuring a pressure in the balloon and an impedance outside the balloon, and feeding back same to a control system; and determining whether the balloon fits with the wall of a blood vessel well. According to the technical solution of the present invention, a pressure in a balloon and an impedance outside the balloon are measured at the same time and compared with set values and set change ranges, such that accurate and automatic determination of an ultrasound ablation balloon fitting with the wall of a blood vessel is achieved, and it is ensured that ablation operation is started after the ultrasound ablation balloon fits with the wall well, thereby greatly improving the ultrasonic transmission efficiency and the ablation operation effect.

## Description

### BACKGROUND

### Technical Field

The present invention relates to an automatic determination method for ultrasound ablation balloon apposition, also relates to an automatic determination system for ultrasound ablation balloon apposition, and belongs to the technical field of medical instruments.

### Related Art

Intravascular ultrasound ablation technology is an interventional procedure method that involves delivering an ultrasound transducer to a designated part in a body and using heat energy generated by ultrasound to ablate a lesion tissue in a target area. The ultrasound transducer is placed inside an ultrasound ablation balloon (referred to as the balloon), and a specific catheter that matches the balloon is used to deliver the balloon to a designated position in the target area. The balloon is filled with an appropriate amount of normal saline to make a diameter of the balloon adapt to a size of a blood vessel in the target area, ensuring good apposition of a surface of the balloon to the wall of the blood vessel. Thus, ablation procedures can be achieved at different positions and different sizes of blood vessels, and the ablation procedure effect is improved.

In existing technology, if no apposition detection is performed on the ultrasound ablation balloon or only a single parameter is used as a reference, it is impossible to accurately determine the apposition of the balloon to the wall of the blood vessel. When the apposition of the balloon to the wall of the blood vessel is poor, ultrasound energy cannot be effectively transmitted to tissues in the target area. This seriously affects the ablation effect. Therefore, it is necessary to develop an automatic determination method for ultrasound ablation balloon apposition.

### SUMMARY

The first technical problem to be solved in the present invention is to provide an automatic determination method for ultrasound ablation balloon apposition.

Another technical problem to be solved in the present invention is to provide an automatic determination system for ultrasound ablation balloon apposition.

To achieve the foregoing objective, the present invention uses the following technical solutions:
According to a first aspect of the embodiments of the present invention, an automatic determination method for ultrasound ablation balloon apposition is provided, including the following steps:
(1) setting related working parameters, where the related working parameters at least include a pressure inside a balloon, a pressure varying range, an impedance outside the balloon, an impedance varying range, and a maximum number of fine adjustments of pressure during apposition determination;
(2) moving a balloon with a built-in ultrasound transducer to a designated position in a target ablation area;
(3) activating a normal saline circulation system to inject normal saline into the balloon, so as to fill and dilate the balloon;
(4) measuring a pressure inside the balloon, and feeding back same to a control system;
(5) performing comparative determination between the measured pressure inside the balloon and a set pressure value; when the pressure inside the balloon reaches the set pressure value, stopping pressurization, and executing a next step; when the pressure inside the balloon does not reach the set pressure value, executing step (3) to continue pressurization, and repeating step (3) to step (5);
(6) measuring an impedance outside the balloon, and feeding back same to the control system;
(7) determining apposition of the balloon; when an apposition condition is satisfied, executing a next step; when the apposition condition is not satisfied, performing fine adjustment of pressure: first accumulating a number of fine adjustments of pressure; when the accumulated number is less than a set maximum number of fine adjustments of pressure, executing step (3), performing fine adjustment of pressure, and repeating step (3) to step (7); when the accumulated number is equal to the set maximum number of fine adjustments of pressure, executing step (9);
(8) starting an ablation operation; and
(9) interrupting and ending the ablation operation in case of an abnormality.

Preferably, the related working parameters set in step (1) further include an ablation area, a temperature of the ablation area, an ultrasound working frequency, ultrasound output power, ablation duration, and sustained duration.

Preferably, in step (7), after the pressure inside the balloon reaches the set pressure value, the impedance outside the balloon is continuously monitored; and when the impedance outside the balloon steadily appears within an impedance varying range set by the control system, the apposition condition is satisfied.

Preferably, when the pressure inside the balloon is measured in step (4), a mean value of a plurality of crest pressure values and trough pressure values before a moment is used as the pressure inside the balloon at the moment.

Preferably, after the ablation operation is started, when the measured pressure inside the balloon exhibits an abnormal sudden change beyond a set pressure value range, and/or the measured impedance outside the balloon exhibits an abnormal sudden change beyond a set impedance range, the control system directly executes step (9).

According to a second aspect of the embodiments of the present invention, an automatic determination system for ultrasound ablation balloon apposition is provided, including an ablation control module, a parameter measurement module, and an ablation balloon module.

The ablation control module is configured to: control configuration of working parameters at beginning of ablation operation, determination of apposition of a balloon to the wall of a blood vessel, and overall monitoring of the process of the ablation operation.

The parameter measurement module is configured to: measure actual values of related working parameters in real time at the beginning of the ablation operation and in the process of the ablation operation, and feed back same to the ablation control module.

A built-in ultrasound transducer of the ablation balloon module is configured to output ultrasound energy to ablate a nervous tissue. At the beginning of the ablation operation, normal saline is injected into the balloon and is used for filling and dilating the balloon to be apposed to the wall of the blood vessel. In the process of the ablation operation, the normal saline is used for implementing conduction of ultrasound energy and cyclic cooling during ablation.

Preferably, during the determination of apposition of the balloon to the wall of the blood vessel, the related working parameters measured by the parameter measurement module at least include a pressure inside the balloon, an impedance outside the balloon, and a number of fine adjustments of pressure during apposition determination.

Preferably, after the pressure inside the balloon reaches a set pressure value, the parameter measurement module continuously monitors the impedance outside the balloon. When the impedance outside the balloon steadily appears within an impedance varying range set by the ablation control module, an apposition condition is satisfied.

Compared with the existing technology, by using the technical solution of simultaneously measuring the pressure inside the balloon and the impedance outside the balloon and comparing same with set values and set varying ranges, the present invention achieves accurate and automatic determination of apposition between an ultrasound ablation balloon and the wall of a blood vessel, and it is ensured that ablation operation is started after the ultrasound ablation balloon is well apposed to the wall, thereby greatly improving the ultrasound transmission efficiency and the ablation procedure effect. Therefore, the automatic determination method for ultrasound ablation balloon apposition provided in the present invention can avoid misjudgment possibly occurring if no apposition detection is performed or if determination is performed only through a single parameter, thus enhancing accuracy of determination of the ultrasound ablation balloon apposition, and the present invention has beneficial effects of high safety of an ablation process, an optimized ablation effect, and the like.

### BRIEF DESCRIPTION OF THE DRAWINGS

FIG. 1 is a schematic diagram of positions and structures of a catheter, a balloon, and an ultrasound transducer of typical ultrasound ablation equipment.
FIG. 2 is a flowchart of an automatic determination method for ultrasound ablation balloon apposition provided in an embodiment of the present invention;
FIG. 3 is a schematic diagram of setting of working parameters of an automatic determination method for ultrasound ablation balloon apposition provided in an embodiment of the present invention; and
FIG. 4 is a block diagram of a structure of an automatic determination system for ultrasound ablation balloon apposition provided in an embodiment of the present invention.

### DETAILED DESCRIPTION

Technical contents of the present invention will be described in detail below with reference to the accompanying drawings and specific embodiments.

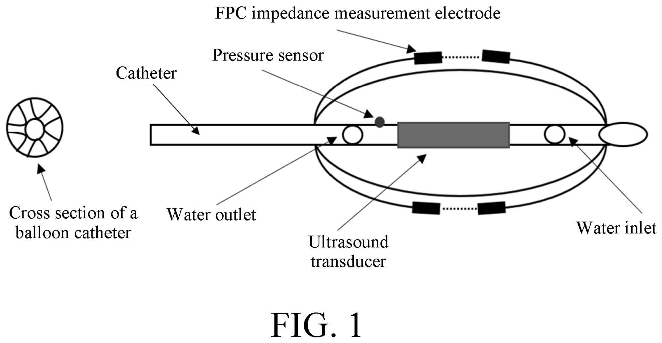

As shown in FIG. 1, in typical ultrasound ablation equipment, a balloon is mounted at one end of a catheter. The ultrasound transducer is located inside the balloon and is fixedly mounted to the catheter. A pressure sensor is disposed within the balloon. An impedance measurement electrode made of a flexible printed circuit (FPC) is disposed on an outer surface of the balloon and is configured to measure a pressure value inside the balloon and an impedance value of an outer of the balloon. A water inlet and a water outlet are respectively formed in a catheter section inside the balloon to inject and circulate normal saline.

When an ultrasound ablation intervention procedure implements ablation inside different blood vessels, it is necessary to fill a flexible ultrasound ablation balloon with an appropriate amount of normal saline to ensure that the flexible ultrasound ablation balloon is apposed to the walls of the blood vessels. The normal saline is used as a transmission medium to deliver ultrasound energy to a target area. Meanwhile, the pressure sensor disposed within the balloon can measure a pressure inside the balloon in real time, and the electrode arranged the outer surface of the balloon can monitor impedance changes in an ablation process in real time.

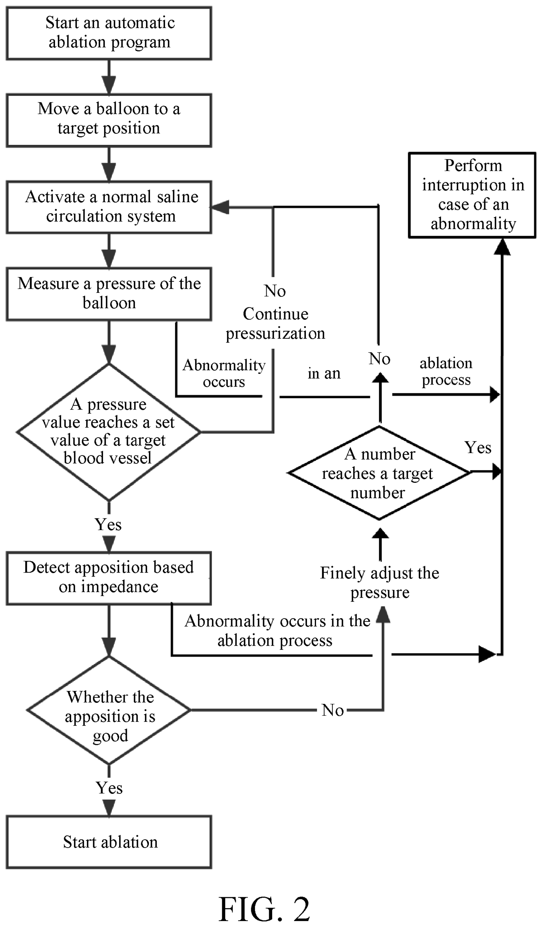

As shown in FIG. 2, an automatic determination method for ultrasound ablation balloon apposition provided in this embodiment of the present invention includes the following steps:
S1: Set related working parameters. The related working parameters at least include a pressure inside a balloon, a pressure varying range, an impedance outside the balloon, an impedance varying range, and a maximum number of fine adjustments of pressure during apposition determination.
S2: Move a balloon with a built-in ultrasound transducer to a designated position in a target ablation area.
S3: Activate a normal saline circulation system to inject normal saline into the balloon, so as to fill and dilate the balloon.
S4: Measure a pressure inside the balloon, and feeding back same to a control system. After ablation operation is started, when the measured pressure inside the balloon exhibits an abnormal sudden change beyond a set pressure value range, step S9 is directly executed.
S5: Perform comparative determination between the measured pressure inside the balloon and a set pressure value; if the pressure inside the balloon reaches the set pressure value, stop pressurization, and execute a next step; if the pressure inside the balloon does not reach the set pressure value, execute step S3 to continue pressurization, and repeat step S3 to step S5.
S6: Measure an impedance value outside the balloon, and feeding back same to the control system. After the ablation operation is started, when the measured impedance outside the balloon exhibits an abnormal sudden change beyond a set impedance value range, step S9 is directly executed.
S7: Determine apposition of the balloon; if an apposition condition is satisfied, execute a next step; if the apposition condition is not satisfied, perform fine adjustment of pressure: first accumulate a number of fine adjustments of pressure; if the accumulated number is less than a set maximum number of fine adjustments of pressure, execute step S3, perform fine adjustment of pressure, and repeat step S3 to step S7; if the accumulated number is equal to the set maximum number of fine adjustments of pressure, execute step S9.
S8: Start the ablation operation.
S9: Interrupt and end the ablation operation in case of an abnormality.

In the above automatic determination method for ultrasound ablation balloon apposition, an ultrasound ablation device involved is configured with a flexible ultrasound ablation balloon. The ultrasound transducer is located inside the balloon and is configured to output ultrasound energy and ablate a nervous tissue at a lesion. The circulated normal saline injected into the balloon is used for conduction of the ultrasound energy and cooling.

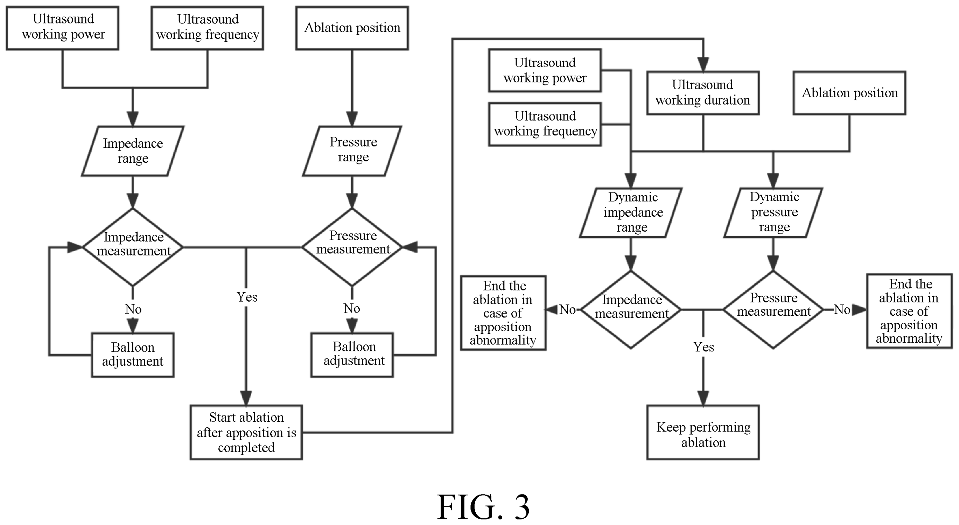

In one embodiment of the present invention, in step S1, the working parameters of this ablation need to be set first. The working parameters include an ablation area, a temperature of the ablation area, an ultrasound working frequency, ultrasound output power, ablation duration, and sustained duration, as well as the pressure inside the balloon, the varying range of the pressure, the impedance outside the balloon, the varying range of the impedance, and the maximum number of fine adjustments of pressure during apposition determination. As shown in FIG. 3, the pressure inside the balloon, the varying range of the pressure, the impedance outside the balloon, the varying range of the impedance, and the maximum number of fine adjustments of pressure during apposition determination are important working parameters for determining the apposition of the balloon to the wall of the blood vessel.

When an ultrasound ablation intervention procedure implements ablation on main trunks or branches at different positions or of different blood vessels, since the blood vessels have different sizes, the pressure inside the balloon and the pressure varying range, which are set by the control system at the beginning, are also different. Moreover, in the ablation process, as the ablation duration of ultrasound energy output increases, the control system can dynamically adjust the pressure inside the balloon and the pressure varying range. Generally, if an inner diameter of the blood vessel is larger, the set pressure inside the balloon and the set pressure varying range are larger.

Corresponding impedances outside the balloon and corresponding impedance varying ranges, which are set by the control system at different ablation positions based on the set ultrasound output power and the set ultrasound working frequency, are also different. Moreover, in the ablation process, since an ion concentration changes because of dehydration of a tissue in the ablation area, the impedance value outside the balloon will gradually decrease. In a normal case, a varying value of the impedance value needs to be within the set impedance varying range. Generally, if a basic impedance value measured before ablation and the ablation duration are larger, the set impedance outside the balloon and the set impedance varying range are larger.

In step S2 and step S3, after the balloon with the built-in ultrasound transducer is moved to the designated position in the target ablation area, the normal saline circulation system is activated to inject the normal saline into the balloon, to fill and dilate the balloon and make the balloon gradually be apposed to the wall of the blood vessel. The normal saline solution injected into the balloon is also used for implementing conduction of ultrasound energy and cyclic cooling during ablation.

In step S4 and step S5, a pressure sensor is disposed within the balloon, the pressure inside the balloon can be measured in real time, and is fed back to the control system. The control system compares the measured pressure inside the balloon with the set pressure value. If the pressure inside the balloon reaches the set pressure value, the normal saline circulation system stops pressurization, maintains the pressure value, ensures that a water outlet velocity and a water inlet velocity of the balloon are consistent, and perform work of the next step. If the measured pressure inside the balloon does not reach the set pressure value, step S3 is executed, and the normal saline circulation system continues pressurization. Then, step S4 and step S5 of measurement of the pressure inside the balloon and pressure determination are repeated until the pressure inside the balloon reaches the set pressure value, and the work of the next step is performed.

After the balloon is apposed to the wall of the blood vessel, the wall of the blood vessel can feed back a pressure to the balloon during the continuation of the pressurization. This feedback pressure can be used for determining whether the pressure inside the balloon has reached the set preset value. Since the pressure inside the balloon placed inside the blood vessel fluctuates with the human pulse, for the measurement of the pressure inside the balloon, a mean value of a plurality of crest pressure values and trough pressure values before a moment is used as the pressure at the moment.

In step S6, when the pressure inside the balloon reaches the set pressure value, the impedance value outside the balloon is measured and fed back to the control system. The measurement of the impedance value outside the balloon is achieved by using an electrode disposed on an outer surface of the balloon. An impedance value measured when the balloon is suspended in blood before it is apposed to the wall of the blood vessel has a significant difference from an impedance value measured after the balloon is apposed to the wall. After the pressure inside the balloon reaches the set pressure value, the impedance value outside the balloon is continuously monitored. If the impedance value outside the balloon steadily appears within an impedance varying range set by the system, the apposition condition is satisfied.

When it is determined in step S7 that an impedance measurement result outside the balloon satisfies the apposition condition, the control system executes to the next step S8 and starts the ablation operation. If the apposition condition is not satisfied, the fine adjustment of pressure is required. In this case, the number of fine adjustments of pressure is first counted or accumulated. If the accumulated number is less than the maximum number set by the control system, step S3 is executed. The normal saline circulation system continues the pressurization for fine adjustment of pressure, and step S4 to step S7 of pressure measurement, pressure determination, impedance measurement, and apposition determination are repeated until the apposition condition is satisfied, and the next step is executed to start the ablation operation. If the apposition condition is still not satisfied when the accumulated number of fine adjustments of pressure reaches the set maximum number, the control system executes step S9, interrupts the apposition operation and the ablation operation, and starts the ablation operation again after an occurring fault is confirmed.

It should be noted that in the ablation process, the impedance value outside the balloon still needs to be measured in real time, so as to monitor the apposition of the balloon to the wall of the blood vessel. If the impedance value outside the balloon exhibits an abnormal sudden change beyond the set range, it indicates that the balloon may not be apposed to the wall or another fault occurs, and the control system immediately interrupts this ablation operation. Similarly, in the ablation process, the pressure inside the balloon still needs to be measured in real time, so as to monitor the apposition of the balloon to the wall of the blood vessel and monitor whether the wall of the blood vessel undergoes severe deformation. When the measured pressure value exhibits an abnormal sudden change beyond the set pressure value range, it indicates that the wall of the blood vessel may undergo unexpected severe deformation. In this case, even if the ablation effect has not been achieved, the control system immediately interrupts the ablation operation.

In addition, the ablation duration set by the control system refers to total duration since the ultrasound transducer starts to operate. In the ablation process, prolonged inflation of the balloon in the blood vessel can obstruct blood flow for a long time, possibly leading to localized tissue ischemia and necrosis. Therefore, when actual ablation duration exceeds the set ablation duration, the ablation operation needs to be interrupted even if the ablation effect has not been achieved.

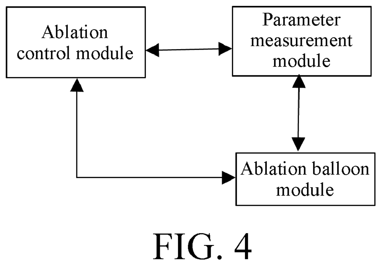

Based on the automatic determination method for ultrasound ablation balloon apposition, the embodiments of the present invention further provide an automatic determination system for ultrasound ablation balloon apposition. As shown in FIG. 4, the automatic determination system for ultrasound ablation balloon apposition includes an ablation control module, a parameter measurement module, and an ablation balloon module.

The ablation control module has a built-in automatic ablation control program and is configured to: control configuration of working parameters at beginning of ablation operation, determination of apposition of a balloon to the wall of a blood vessel, and overall monitoring of a process of the ablation operation. At the beginning of the ablation operation, specific working parameters are first configured and set based on an ablation area and an ablation purpose. The working parameters at least include the ablation area, a temperature of the ablation area, an ultrasound working frequency, ultrasound output power, ablation duration, and sustained duration, as well as a pressure inside the balloon, a pressure varying range, an impedance outside the balloon, an impedance varying range, and a maximum number of fine adjustments of pressure during apposition determination. Second, after a balloon with a built-in ultrasound transducer is moved to a designated position in a target ablation area, a circulation system is controlled to inject normal saline into the balloon until the pressure inside the balloon reaches a set target value. Then, the circulation system determines whether the balloon and the blood vessel satisfy an apposition condition. In addition, in the process of the ablation operation, feedback information of the parameter measurement module is received, the related working parameters are adjusted, and an ablation progress is controlled.

The parameter measurement module is configured to: measure actual values of related working parameters in real time at the beginning of the ablation operation and in the process of the ablation operation, and feed back same to the ablation control module. The related working parameters measured at least include a pressure inside the balloon, an impedance outside the balloon, a number of fine adjustments of pressure during apposition determination, and the like. At the beginning of the ablation operation, when it is measured that the number of fine adjustments of pressure during apposition determination exceeds a set maximum number, or in the process of the ablation operation, when it is measured that the pressure inside the balloon and/or the impedance outside the balloon exhibits an abnormal sudden change, same is fed back to a control system to forcibly interrupt the apposition operation or the ablation operation, thus ensuring safety of an ablation intervention procedure.

A built-in ultrasound transducer of the ablation balloon module is configured to output ultrasound energy to ablate a nervous tissue. At the beginning of the ablation operation, normal saline is injected into the balloon and is used for filling and dilating the balloon to be apposed to the wall of the blood vessel. In the process of the ablation operation, the normal saline is used for implementing conduction of ultrasound energy and cyclic cooling during ablation. The above three modules work together to implement automatic determination on ultrasound ablation balloon apposition.

It should be noted that the control system in this embodiment of the present invention can be implemented by a micro processor or a microcontroller unit with a built-in automatic ablation control program. In some embodiments, the control system can also serve as the above ablation control module.

In conclusion, compared with the existing technology, by using the technical solution of simultaneously measuring the pressure inside the balloon and the impedance outside the balloon and comparing same with set values and set varying ranges, the automatic determination method for ultrasound ablation balloon apposition provided in this embodiment of the present invention achieves accurate and automatic determination of apposition between an ultrasound ablation balloon and the wall of a blood vessel, and it is ensured that ablation operation is started after the ultrasound ablation balloon is well apposed to the wall, thereby greatly improving the ultrasound transmission efficiency and the ablation procedure effect. Therefore, the automatic determination method for ultrasound ablation balloon apposition provided in this embodiment of the present invention can avoid misjudgment possibly occurring if no apposition detection is performed or if determination is performed only through a single parameter, thus enhancing accuracy of determination of the ultrasound ablation balloon apposition, and the present invention has beneficial effects of high safety of an ablation process, an optimized ablation effect, and the like.

It should be noted that the plurality of embodiments described above are for illustrative purpose only. The technical solutions of the embodiments may be combined, all of which fall within the protection scope of the present invention.

In addition, the terms "first" and "second" are used for descriptive purposes only and are not to be construed as indicating or implying relative importance or to implicitly indicate the number of technical features indicated. From this, features defined as "first" and "second" may explicitly or implicitly include one or more features. In the description of the present invention, "plurality" means two or more, unless otherwise expressly and specifically defined.

The above has described in detail the automatic determination method and system for ultrasound ablation balloon apposition provided in the present invention. For those of ordinary skill in the art, any obvious changes made to the present invention without deviating from its substantive content will constitute infringement of the patent rights of the present invention, and they will bear corresponding legal responsibilities.

## Claims

1. An automatic determination method for ultrasound ablation balloon apposition, comprising the following steps:
(1) setting related working parameters, wherein the related working parameters at least comprise a pressure inside a balloon, a pressure varying range, an impedance outside the balloon, an impedance varying range, and a maximum number of fine adjustments of pressure during apposition determination;
(2) moving a balloon with a built-in ultrasound transducer to a designated position in a target ablation area;
(3) activating a normal saline circulation system to inject normal saline into the balloon, so as to fill and dilate the balloon;
(4) measuring a pressure inside the balloon, and feeding back same to a control system;
(5) performing comparative determination between the measured pressure inside the balloon and a set pressure value; when the pressure inside the balloon reaches the set pressure value, stopping pressurization, and executing a next step; when the pressure inside the balloon does not reach the set pressure value, executing step (3) to continue pressurization, and repeating step (3) to step (5);
(6) measuring an impedance outside the balloon, and feeding back same to the control system;
(7) determining apposition of the balloon; when an apposition condition is satisfied, executing a next step; when the apposition condition is not satisfied, performing fine adjustment of pressure: first accumulating a number of fine adjustments of pressure; when the accumulated number is less than a set maximum number of fine adjustments of pressure, executing step (3), performing fine adjustment of pressure, and repeating step (3) to step (7); when the accumulated number is equal to the set maximum number of fine adjustments of pressure, executing step (9);
(8) starting an ablation operation; and
(9) interrupting and ending the ablation operation in case of an abnormality.

2. The automatic determination method for ultrasound ablation balloon apposition according to claim 1, wherein
the related working parameters set in step (1) further comprise an ablation area, a temperature of the ablation area, an ultrasound working frequency, ultrasound output power, ablation duration, and sustained duration.

3. The automatic determination method for ultrasound ablation balloon apposition according to claim 1, wherein
in step (7), after the pressure inside the balloon reaches the set pressure value, the impedance outside the balloon is continuously monitored; and when the impedance outside the balloon steadily appears within an impedance varying range set by the control system, the apposition condition is satisfied.

4. The automatic determination method for ultrasound ablation balloon apposition according to claim 1, wherein
when the pressure inside the balloon is measured in step (4), a mean value of a plurality of crest pressure values and trough pressure values before a moment is used as the pressure inside the balloon at the moment.

5. The automatic determination method for ultrasound ablation balloon apposition according to claim 1, wherein
after the ablation operation is started, when the measured pressure inside the balloon exhibits an abnormal sudden change beyond a set pressure value range, and/or the measured impedance outside the balloon exhibits an abnormal sudden change beyond a set impedance range, the control system directly executes step (9).

6. An automatic determination system for ultrasound ablation balloon apposition, comprising an ablation control module, a parameter measurement module, and an ablation balloon module, wherein
the ablation control module is configured to: control configuration of working parameters at beginning of ablation operation, determination of apposition of a balloon to the wall of a blood vessel, and overall monitoring of a process of the ablation operation;
the parameter measurement module is configured to: measure actual values of related working parameters in real time at the beginning of the ablation operation and in the process of the ablation operation, and feed back same to the ablation control module;
an ultrasound transducer is disposed within the ablation balloon module and is configured to output ultrasound energy to ablate a nervous tissue; at the beginning of the ablation operation, normal saline is injected into the balloon and is used for filling and dilating the balloon to be apposed to the wall of the blood vessel; and in the process of the ablation operation, the normal saline is used for implementing conduction of ultrasound energy and cyclic cooling during ablation.

7. The automatic determination system for ultrasound ablation balloon apposition according to claim 6, wherein
during the determination of apposition of the balloon to the wall of the blood vessel, the related working parameters measured by the parameter measurement module at least comprise a pressure inside the balloon, an impedance outside the balloon, and a number of fine adjustments of pressure during apposition determination.

8. The automatic determination system for ultrasound ablation balloon apposition according to claim 6, wherein
after the pressure inside the balloon reaches a set pressure value, the parameter measurement module continuously monitors the impedance outside the balloon; and when the impedance outside the balloon steadily appears within an impedance varying range set by the ablation control module, an apposition condition is satisfied.
